# EUROPEAN PATENT SPECIFICATION

(11) **EP 2 859 835 B1**
(45) Date of publication and mention of the grant of the patent: **28.06.2017**
(21) Application number: 14185551.0
(22) Date of filing: 19.09.2014
(51) Int. Cl.: A61B 1/00, A61B 1/015, A61B 1/12

(54) **Endoscope**
Endoskop
Endoscope

(30) Priority: 08.10.2013 JP 2013211144
(43) Date of publication of application: 15.04.2015
(73) Proprietor: Fujifilm Corporation, Minato-ku Tokyo 106-8620 (JP)
(72) Inventor: Ikeda, Toshiyuki, Kanagawa (JP); Torii, Yuichi, Kanagawa (JP); Iyama, Shozo, Kanagawa (JP)
(74) Representative: Klunker IP Patentanwälte PartG mbB

(56) References cited:
- EP-A1- 1 665 978
- EP-A1- 2 491 848
- EP-A1- 2 494 909
- JP-A- 2010 279 533
- US-A1- 2007 027 360
- US-A1- 2011 112 363

## Description

### BACKGROUND OF THE INVENTION

### 1. Field of the Invention

The present invention relates to an endoscope having a fluid ejection nozzle for ejecting fluid to an observation window.

### 2. Description Related to the Prior Art

An endoscope is used in a medical examination and the like of a digestive system, for example. This endoscope is provided at a distal end of an insert section to be introduced into a human body cavity, with an observation window for taking image light of an object, lighting windows for applying illumination light to the object, and a fluid ejection nozzle (an air and water nozzle) for ejecting fluid (water or gas such as air or carbonic acid gas) to the observation window. Since a surface of the observation window gets soiled with body fluid and dirt, water is ejected from an ejection port of the fluid ejection nozzle to wash the observation window, and then air is ejected from the ejection port to blow water droplets off the surface of the observation window. The dirt or the water droplets remaining on a part of the observation window interfere with observation, so it is desirable that the fluid ejected from the fluid ejection nozzle reach all over the surface of the observation window.

According to endoscopes described in Japanese Patent Laid-Open Publication Nos. 2011-120863 and 2012-179221, a surface of an observation window is disposed so as to protrude from a flat surface of a distal portion of an insert section by a predetermined height, and a slope whose height is gradually increased from the flat surface to the surface of the observation window is formed along the rim of the observation window. Fluid ejected from an ejection port of a fluid ejection nozzle runs into the slope, and smoothly flows to the surface of the observation window. Therefore, the fluid can reach all over the surface of the observation window. JP 2010-279533 discloses a device according to the preamble of claim 1. In the endoscopes of the above Japanese Patent Laid-Open Publication Nos. 2011-120863 and 2012-179221, the fluid ejected from the fluid ejection nozzle has high flow velocity in the vicinity of the middle of a nozzle opening, while has low flow velocity in the vicinity of the periphery of the nozzle opening. Especially, in the case of selectively ejecting liquid and gas from the same fluid ejection nozzle, an ejection area differs depending on difference in viscosity, specific gravity, and the like between the liquid and the gas. For example, the liquid tends to have a narrow ejection area less spreading in a lateral direction, while the gas tends to have a wide ejection area spreading in the lateral direction. Therefore, on the occasion of blowing off the water droplets remaining on the observation window by ejection of the gas, after the observation window is washed with the liquid e. g. water ejected from the liquid ejection nozzle, a high-flow-velocity area in the vicinity of the middle of a gas flow ejected from the fluid ejection nozzle reliably blows moisture off the observation window. However, in a peripheral low-flow-velocity area, moisture remaining on a distal surface is drifted and moved as water droplets, and builds up on the lighting windows. Especially, the farther from a tip of the nozzle, the more conspicuous the build-up of the water droplets becomes.

The build-up of the water droplets on the lighting windows causes halation, flare, and the like, and deteriorates an endoscopic image. Also, in a recent endoscope whose angle of view field tends to widen, the water droplets are seen in the view field and narrow the view field thereby. To prevent the build-up of the water droplets on the flat surface of the distal portion, the observation window and the lighting windows may be disposed apart. In this case the water droplets pass through between the observation window and the lighting window and move out of the flat surface. Even if the water droplets remain on the flat surface, the water droplets are present outside the view field and hardly interfere with the observation image. However, it is desired that the insert section including the distal portion have a small diameter for the purpose of reducing a burden to a human body. On the other hand, it is also desired with improvement in image quality of the observation image that the observation window and the lighting windows have large diameters. For these reasons, close disposition of the observation window and the lighting windows cannot be helped, and leaving no water droplet is desired in consideration of these circumstances.

### SUMMARY OF THE INVENTION

An object of the present invention is to provide an endoscope in which water droplets building up at a distal portion can be reliably removed.

To achieve the above and other objects, an endoscope according to the present invention includes a flat surface, an observation window, a fluid ejection nozzle, a lighting window, a suction port, and a fluid guide slope. The flat surface is formed in a distal portion of an insert section to be introduced into a body cavity in an orthogonal manner to an axial direction of the insert section. The observation window is disposed in the flat surface, for observing the inside of the body cavity. The fluid ejection nozzle is disposed on the flat surface, for ejecting fluid onto a surface of the observation window. The lighting window is positioned in an ejection area of the fluid ejected from the fluid ejection nozzle and disposed in the flat surface adjacently to the observation window, for applying illumination light to illuminate the inside of the body cavity. The suction port is disposed in the flat surface adjacently to the lighting window, the observation window, and the fluid ejection nozzle, for sucking the fluid. The fluid guide slope is disposed in the ejection area of the fluid between the lighting window and the suction port so as to connect the flat surface to the suction port in an inclined manner relative to the flat surface. The length of the fluid guide slope from the flat surface to the suction port is increased with distance from the fluid ejection nozzle.

It is preferable that the fluid ejection nozzle selectively eject gas and liquid, and an ejection area of the gas be wider in a lateral direction than an ejection area of the liquid. The observation window is preferably disposed in the ejection area of the liquid, and the lighting window is preferably disposed in the ejection area of the gas.

The fluid guide slope is preferably the deepest relative to the flat surface at a surface in a line connecting the center of the suction port to the center of the lighting window positioned in the ejection area of the fluid. Also, the fluid guide slope is preferably formed so as to enlarge the internal diameter of the suction port from the center of the suction port to the center of the lighting window.

The fluid guide slope is preferably positioned at least in an area connecting the center of the suction port, the center of the lighting window, and the center of the observation window. The distal portion preferably includes a distal portion body and a distal end cap attached to a tip of the distal portion body. The fluid guide slope is preferably formed in the distal end cap.

The fluid guide slope is preferably formed along an edge of the ejection area of the liquid ejected from the fluid ejection nozzle.

The fluid ejection nozzle and the suction port are preferably adjacent to each other. The lighting window is preferably disposed opposite to the ejection port of the fluid ejection nozzle beyond the fluid guide slope in a fluid ejection direction of the fluid ejection nozzle.

It is preferable that a tapered surface be formed along the rim of the observation window and the diameter of the tapered surface be gradually reduced from the flat surface to the observation window. The tapered surface and the fluid guide slope are preferably formed continuously. It is also preferable that the tapered surface be formed along the rim of the observation window and the diameter of the tapered surface be gradually reduced from the flat surface to the observation window, and the tapered surface and the lighting window be adjacent to each other.

It is preferable that there be a plurality of the lighting windows, and the fluid guide slope be disposed between the suction port and the lighting window that is the farthest from the fluid ejection nozzle and the nearest to the suction port. The suction port preferably functions as a forceps outlet too.

The endoscope according to the present invention has the fluid guide slope that is disposed between the lighting window and the suction port in the ejection area of the fluid. The fluid guide slope connects the flat surface to the suction port in an inclined manner relative to the flat surface. The length of the fluid guide slope from the flat surface to the suction port is gradually increased with distance from the fluid ejection nozzle. Therefore, it is possible to reliably remove water droplets building up on the flat surface of the distal portion or moving during supplying the gas.

### BRIEF DESCRIPTION OF DRAWINGS

For more complete understanding of the present invention, and the advantage thereof, reference is now made to the subsequent descriptions taken in conjunction with the accompanying drawings, in which:
Fig. 1 is an external perspective view of an endoscope system;
Fig. 2 is a conduit diagram of an electronic endoscope;
Fig. 3 is a perspective view showing the structure of a distal portion of the electronic endoscope;
Fig. 4 is a cross sectional view taken along line IV-IV in Fig. 3;
Fig. 5 is a plan view showing a state of ejecting fluid onto an observation window;
Fig. 6 is a plan view showing an example of disposition of a slope in a part that tends to have water droplets;
Fig. 7 is a perspective view showing a distal portion of an insert section according to a second embodiment in which a tapered surface and a fluid guide slope are continued; and
Fig. 8 is a cross sectional view taken along line VIII-VIII in Fig. 7.

### DESCRIPTION OF THE PREFERRED EMBODIMENTS

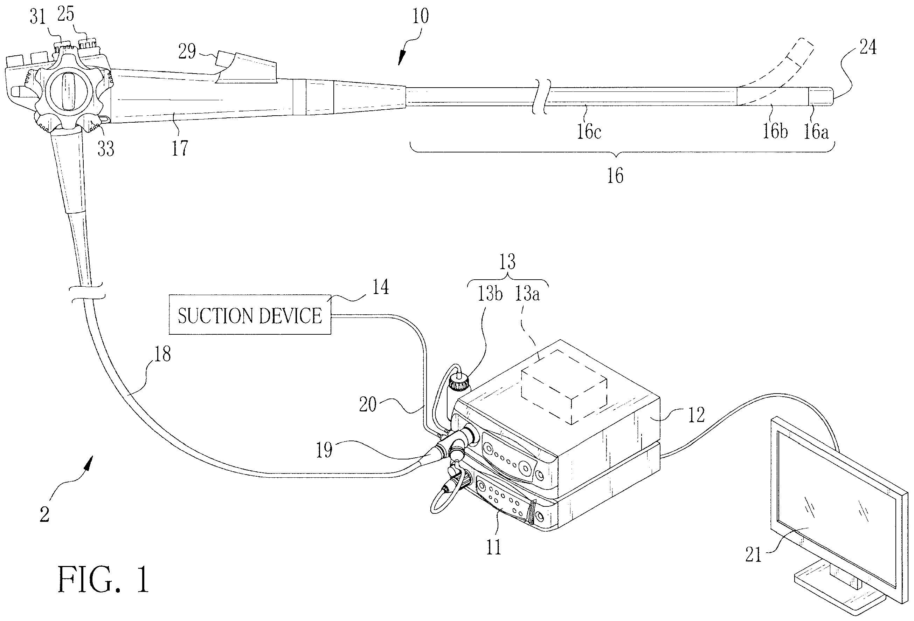

As shown in Fig. 1, an endoscope 2 is constituted of an electronic endoscope 10, a processor device 11, a light source device 12, an air and water supply device 13, and a suction device 14. The air and water supply device 13 has a well-known air supply device (pump or the like) 13a, which is contained in the light source device 12 and supplies gas, and a wash water tank 13b, which is provided outside of the light source device 12 and contains wash water. The electronic endoscope 10 includes a flexible insert section 16 to be introduced into a human body cavity, a handling section 17 coupled to a proximal part of the insert section 16, and a universal cord 18 connected to the processor device 11 and the light source device 12.

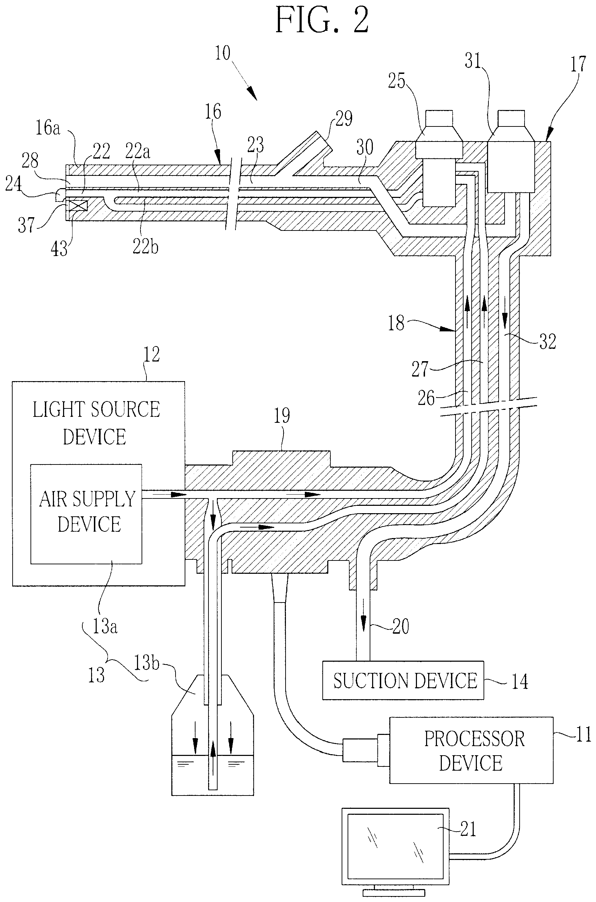

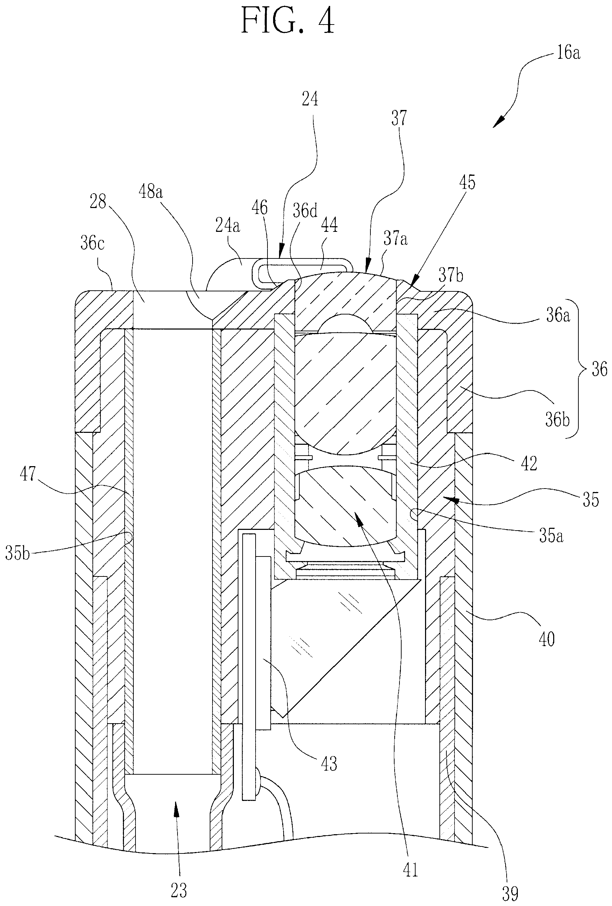

The insert section 16 has a distal portion 16a provided at a distal end thereof, a bending portion 16b coupled to a proximal end of the distal portion 16a, and a flexible tube portion 16c coupled to a proximal end of the bending portion 16b. The distal portion 16a contains a CCD image sensor (see Figs. 2 and 4, hereinafter called CCD) 43, as an imaging device for imaging the inside of the body cavity, and the like. Hereinafter, a distal side of the insert section 16 is simply referred to as "distal side", and a proximal side of the insert section 16 is simply referred to as "proximal side".

The universal cord 18 is attached with a connector 19 at its distal end. The connector 19, being a multi connector, is connected to the processor device 11, the light source device 12, and the air and water supply device 13. A suction device 14 is also connected to the connector 19 through a connection tube 20.

The processor device 11 is electrically connected to the light source device 12, and performs centralized control of the operation of the endoscope system 2. The processor device 11 feeds power to the electronic endoscope 10 through a transmission cable, which extends through the universal cord 18 and the insert section 16, and controls the operation of the CCD 43. Also, the processor device 11 obtains an imaging signal outputted from the CCD 43 through the transmission cable, and produces image data by applying various types of image processing to the imaging signal. The image data produced by the processor device 11 is displayed as an observation image on a monitor 21 connected to the processor device 11 through a cable.

As shown in Fig. 2, an air and water supply channel 22 and a forceps channel 23 are disposed inside the insert section 16 and the handling section 17. One end of the air and water supply channel 22 is coupled to an air and water nozzle (fluid ejection nozzle) 24 provided in the distal portion 16a. The other end of the air and water supply channel 22 is branched into an air supply conduit 22a and a water supply conduit 22b. The air supply conduit 22a and the water supply conduit 22b are connected to an air and water supply button 25 provided on the handling section 17.

To the air and water supply button 25, one end of an air source conduit 26 extending to the air supply device 13a and one end of a water source conduit 27 extending to the wash water tank 13b are connected, in addition to the air supply conduit 22a and the water supply conduit 22b. The air supply device 13a supplies gas (air or carbon acid gas) during endoscopy with the electronic endoscope 10.

Upon performing air supply operation by operating the air and water supply button 25, the gas produced by the air supply device 13a is sent to the air and water nozzle 24. Upon performing water supply operation by operating the air and water supply button 25, the wash water is sent from the wash water tank 13b to the air and water nozzle 24 by pressure of the gas produced by the air supply device 13a. The air and water nozzle 24 selectively ejects the gas and the wash water supplied through the air and water supply channel 22.

The forceps channel 23 is coupled to a forceps outlet (suction port) 28 at one end, and connected to a forceps inlet 29 at the other end. Into the forceps inlet 29, are inserted one or more of various types of medical instruments having an injection needle, a high-frequency knife, and the like at their tips. Except when the medical instrument is inserted, the forceps inlet 29 is clogged with a lid (not shown). From the forceps channel 23, a suction conduit 30 branches off. This suction conduit 30 is connected to a suction button 31.

Not only the suction conduit 30 but also one end of a suction source conduit 32 is connected to the suction button 31. The other end of the suction source conduit 32 leads to the suction device 14 through the connection tube 20. The suction device 14 is always kept operated during endoscopy with the electronic endoscope 10. Upon performing suction operation by operating the suction button 31, suction is performed by negative pressure produced by the suction device 14. Upon interrupt operation, the suction is stopped by interrupting the negative pressure.

The handling section 17 is provided with the forceps inlet 29, the air and water supply button 25, the suction button 31, an angle knob 33, and the like. Operating the angle knob 33 bends the bending portion 16b up and down and side to side by pushing and pulling wires inserted through the insert section 16. Thus, the distal portion 16a is aimed at a desired direction inside the body cavity.

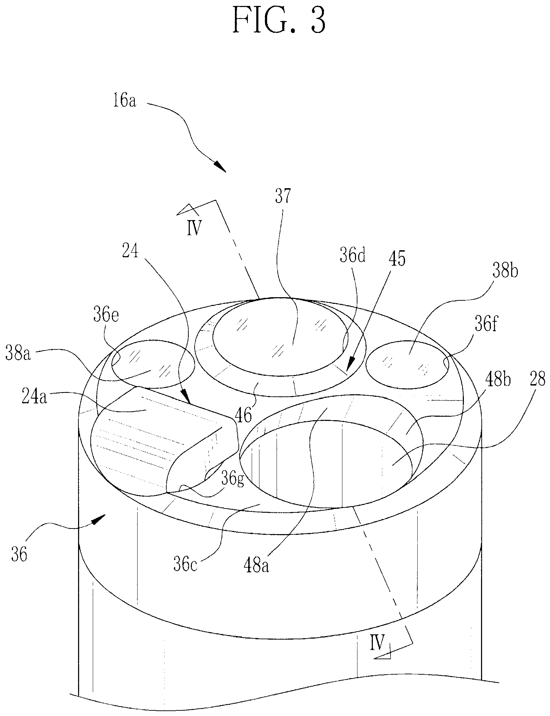

As shown in Figs. 3 to 5, the distal portion 16a includes a distal portion body 35, a distal end cap 36 attached to a tip of the distal portion body 35, an observation window 37, two lighting windows 38a and 38b, the air and water nozzle 24, and the forceps outlet 28. The distal portion body 35 is formed with through holes 35a, 35b, and the like along an axial direction of the insert section 16, for holding the air and water nozzle 24 and after-mentioned parts including an objective lens unit 41, a connection pipe 47, a light guide, and the like. A rear end of the distal portion body 35 is coupled to a bending piece 39 at a distal end of the bending portion 16b. Note that, in Fig. 4, the thickness of each part in a radial direction is exaggerated for the purpose of illustration. Accordingly, the diameters of the forceps outlet 28 and the observation window 37 are illustrated smaller than the actual diameters. Fig. 4 does not agree with the perspective view of Fig. 3 in thickness.

The distal end cap 36 has a distal end plate portion 36a for covering an end of the distal portion body 35, and a cylindrical portion 36b for covering the periphery of the distal portion body 35. A sheath layer 40 for covering the periphery of the bending portion 16b extends to the distal portion body 35. A front end of the sheath layer 40 contacts and is secured to a rear end of the cylindrical portion 36b with an adhesive or the like. The distal end plate portion 36a is formed with a flat surface 36c, which is a surface orthogonal to the axial direction of the insert section 16 and composes a distal end surface of the insert section 16. Note that, the "orthogonal" surface described here includes a surface approximately orthogonal to the axial direction of the insert section 16.

Viewing the flat surface 36c from a distal end side, the distal end plate portion 36a is formed with the forceps outlet 28 and through holes 36d to 36g, which expose the observation window 37, the lighting windows 38a and 38b, and the air and water nozzle 24, respectively.

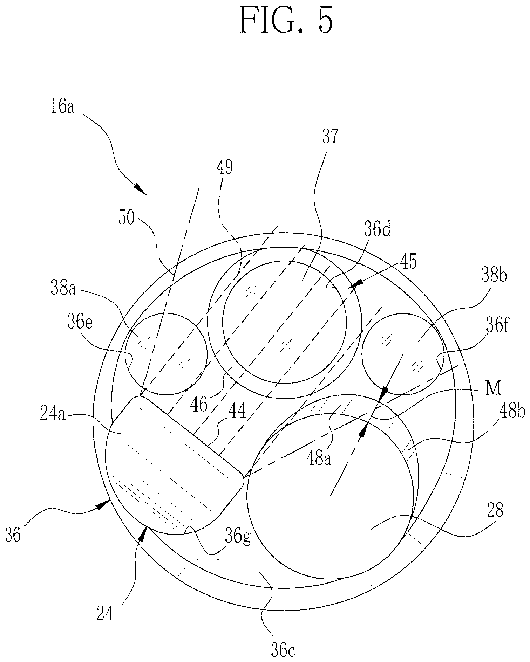

Note that, a perpendicular direction in Fig. 5 corresponds to a perpendicular direction of bending of the bending portion 16b. The observation window 37 is disposed in an upper part of the flat surface 36c, and the air and water nozzle 24 and the forceps outlet 28 are disposed under the observation window 37. The lighting windows 38a and 38b are disposed symmetrically with respect to the observation window 37.

The observation window 37 is an objective lens at the most distal end of the objective lens unit 41, and also functions as a cover glass. The outside of the observation window 37 is in a disc shape. A surface 37a of the observation window 37, being a light incident surface, is formed in a convex lens or flat, and is the convex lens in this embodiment. An optical system of the objective lens unit 41 including the observation window 37 is held by a lens barrel 42. The lens barrel 42 holds a proximal end side of a periphery 37b of the observation window 37. A distal end side of the periphery 37b of the observation window 37 is fitted into the through hole 36d of the distal end cap 36.

The lens barrel 42 is fitted into the through hole 35a of the distal portion body 35, and disposed in such a position that a front end surface of the lens barrel 42 contacts the distal end plate portion 36a of the distal end cap 36 and the observation window 37 is exposed from a distal end side of the flat surface 36c. As described above, the observation window 37 is attached such that the surface 37a is coplanar to or protrudes from the flat surface 36c. In this embodiment, the observation window 37 is attached in such a position that the surface 37a protrudes from the flat surface 36c. The surface 37a of the observation window 37 protrudes from the flat surface 36c by 0.1 to 0.3 mm, for example.

Note that, the observation window 37 may be a cover glass that is at the most distal end of the objective lens unit 41 and has no lens effect. Otherwise, the observation window 37 may not constitute the objective lens unit 41, and be securely fitted into the through hole 36d of the distal end cap 36 as a simple cover glass.

As shown in Fig. 4, the CCD 43 is attached in the deep recess of the objective lens unit 41. The CCD 43, being an interline transfer type CCD, for example, forms an image of an object captured by the optical system of the objective lens unit 41 on its imaging surface. Note that, the imagine device is not limited to the CCD 43, but may be a CMOS image sensor or another image sensor.

The lighting windows 38a and 38b also function as lighting lenses, and apply illumination light from the light source device 12 to an observation portion inside the human body cavity. Light exit ends of the light guide (not shown) are opposed to the lighting windows 38a and 38b. The light guide is made of a bundle of many optical fibers. This light guide extends through the insert section 16, the handling section 17, the universal cord 18, and the connector 19, and leads the illumination light from the light source device 12 to the lighting windows 38a and 38b.

The air and water nozzle 24 is connected to the air and water supply channel 22 by being fitted into the periphery of a distal end of the air and water supply channel 22 at its proximal end. The proximal end of the air and water nozzle 24 and the air and water supply channel 22 are fitted into the through hole 36g of the distal portion body 35. The air and water nozzle 24 is formed with an ejection tube 24a on its distal end side. The ejection tube 24a is formed in the shape of a tube bent into an approximately 90° smoothly from the distal end of the air and water nozzle 24 to an ejection port 44 at a distal end. The ejection tube 24a is exposed outside through the through hole 36g of the distal end cap 36, and disposed on the flat surface 36c.

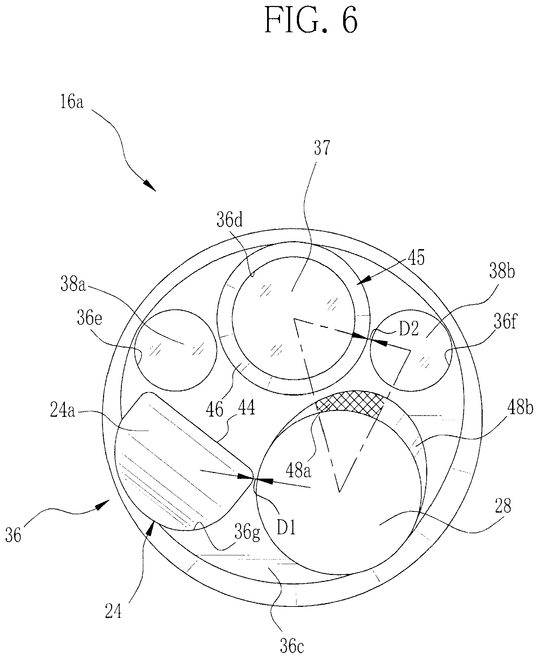

The air and water nozzle 24 and the forceps outlet 28 are adjacent to each other. Note that, the "adjacent" disposition of the air and water nozzle 24 and the forceps outlet 28 described here refers to a condition in which the periphery of the air and water nozzle 24 and the circumference of the forceps outlet 28 are adjacently disposed at a distance D1 (see Fig. 6) between or equal to 0 mm and 0.5 mm, for example.

As shown in Fig. 5, out of fluid ejected from the air and water nozzle 24, wash water 49 is linearly blown on the observation window 37, as shown by dashed lines in Fig. 5. Gas 50 is blown so as to spread from the air and water nozzle 24 to an area shown by alternate long and short dashed lines in Fig. 5. The observation window is disposed within an ejection area of the wash water ejected from the air and water nozzle 24. The fluid ejected from the air and water nozzle 24, either of the wash water and the gas, has high flow velocity in the vicinity of the middle of the ejection port 44, while has low flow velocity in the vicinity of the periphery of the ejection port 44.

The distal end cap 36 is integrally formed with an annular convex portion 45, which protrudes from the flat surface 36c by a predetermined height, between the rim of the observation window 37 and the flat surface 36c. The inner periphery of the annular convex portion 45 is continued to the through hole 36d, and the outer periphery of the annular convex portion 45 is formed with a tapered surface 46. The tapered surface 46 is formed over the entire rim of the observation window 37 such that a diameter is gradually reduced to the observation window 37.

The lighting windows 38a and 38b are adjacent to the observation window 37 and coplanar to the flat surface 36c, and disposed in an ejection area of the gas ejected from the air and water nozzle 24. One lighting window 38b is adjacent to the forceps outlet 28, and far from the air and water nozzle 24 beyond the observation window 37. The other lighting window 38a is far from the forceps outlet 28 beyond the observation window 37, and adjacent to the air and water nozzle 24. The lighting windows 38a and 38b are adjacent to the tapered surface 46. Note that, the "adjacent" disposition of the lighting windows 38a and 38b and the tapered surface 46 described here refers to a condition in which the rim of each lighting window 38a, 38b and the circumference of the tapered surface 46 are adjacent at a distance D2 (see Fig. 6) between or equal to 0.1 mm and 0.3 mm, for example.

The forceps outlet 28 is connected to the forceps channel 23 through the connection pipe 47 fitted into the through hole 35b of the distal portion body 35, and leads to the forceps inlet 29. Each of the various types of medical instruments is inserted into the forceps inlet 29, and a tip of the medical instrument is exposed from the forceps outlet 28. The suction conduit 30 is connected to the forceps channel 23, as described above, so that the forceps outlet 28 also functions as a suction port for sucking the fluid.

In the distal end cap 36, fluid guide slopes 48a and 48b are formed between the lighting window 38b and the forceps outlet 28. The fluid guide slops 48a and 48b are inclined with respect to the flat surface 36c, and connect the flat surface 36c to the forceps outlet 28. The fluid guide slope 48a is disposed on the side of the air and water nozzle 24 with respect to a line connecting the center of the lighting window 38b to the center of the forceps outlet 28, and the fluid guide slope 48b is disposed on the side of the periphery of the distal end cap 36.

The fluid guide slope 48a is disposed in the ejection area of the gas ejected from the air and water nozzle 24 along an edge of the ejection area of the wash water ejected from the air and water nozzle 24. The fluid guide slopes 48a and 48b are formed in the shape of a crescent in which the length from the flat surface 36c to the forceps outlet 28 is gradually increased with distance from the air and water nozzle 24. The fluid guide slope 48a is smoothly continued to the edge of the forceps outlet 28 at an end portion on the side of the air and water nozzle 24. A surface in a line connecting the center of the forceps outlet 28 to the center of the lighting window 38b is the deepest from the flat surface 36c. To be more specific, the length M of the fluid guide slope 48a is maximized in a direction of the line connecting the center of the forceps outlet 28 to the center of the lighting window 38b. This length M is between or equal to 0.3 mm and 0.5 mm.

Also, as shown in Fig. 6, the fluid guide slope 48a is situated in an area to which the water droplets ejected from the air and water nozzle 24 tend to adhere or move. To be more specific, a part (a hatched portion) of the fluid guide slope is situated at least within an area connecting the center of the forceps outlet 28, the center of the lighting window 38b, and the center of the observation window 37. Furthermore, the fluid guide slope 48a is disposed between the observation window 37 and the lighting window 38b, and the lighting window 38b is disposed in a position opposite to the ejection port 44 of the air and water nozzle 24 beyond the fluid guide slope 48a in a fluid ejection direction of the air and water nozzle 24.

The fluid guide slope 48b is continued to the fluid guide slope 48a at the position of maximizing the length M. The length of the fluid guide slope 48b is gradually reduced with distance from the air and water nozzle 24. The fluid guide slope 48b is smoothly continued to the edge of the forceps outlet 28 at an end on the side of the periphery of the distal end cap 36. As described above, the fluid guide slopes 48a and 48b are formed so as to enlarge the internal diameter of the forceps outlet 28 from the center of the forceps outlet 28 to the center of the lighting window 38b.

A process of washing the observation window 37 by ejecting the fluid from the air and water nozzle 24 will be described. As described above, the observation window 37 and the tapered surface 46 are situated in the fluid ejection area of the air and water nozzle 24. Out of the fluid (wash water or gas) ejected from the air and water nozzle 24, a part of the wash water is directly blown onto the observation window 37. The remaining wash water bumps against the tapered surface 46 and expands in a circumferential direction of the observation window 37, and climbs up the tapered surface 46. Thus, the wash water is distributed over the entire surface of the observation window 37, so that body fluid and dirt adhering to the observation window 37 are blown off. Furthermore, ejection of the gas blows the wash water too.

Out of the fluid ejected from the air and water nozzle 24, the wash water ejected from the vicinity of the periphery of the ejection port 44 has low flow velocity. Accordingly, water droplets are sometimes built up on the flat surface 36c in the vicinity of the air and water nozzle 24 just moments before completing ejection of the wash water. Moreover, after the ejection of the wash water, upon starting ejection of the gas, the wash water remaining in the vicinity of the air and water nozzle 24 is sometimes built up on the flat surface 36c as water droplets. These water droplets are certainly blown off in a high-flow-velocity area in the vicinity of the middle of a gas flow ejected from the air and water nozzle 24. However, in a low-flow-velocity area in the periphery of the gas flow, the water droplets are not blown off and move toward the lighting window 38b, and are built up again. However, since the fluid guide slope 48a is situated in the ejection area of the gas ejected from the air and water nozzle 24 along the edge of the ejection area of the wash water ejected from the air and water nozzle 24, the built-up or moved water droplets flow along the fluid guide slope 48a. Especially, the water droplets built up in the area connecting the center of the observation window 37, the center of the lighting window 38a, and the center of the forceps outlet 28 easily flow into the fluid guide slope 48a. Also, by operating the suction button 31, the water droplets adhering to the fluid guide slope 48a or built up in the vicinity of the lighting window 38b can be led to the fluid guide slope 48a or 48b and removed by suction. On the other hand, the lighting window 38a is nearer to the air and water nozzle 24 than the lighting window 38b. Almost all water droplets built up in the vicinity of the lighting window 38a are within the high-flow-velocity area of the gas flow ejected from the air and water nozzle 24, and hence blown off by the gas ejected from the air and water nozzle 24.

As described above, providing the fluid guide slope 48a in the distal portion 16a allows reliable removal of the built-up or moved water droplets. Therefore, it is possible to prevent the occurrence of halation, flare, and the like, and hence prevent deterioration in an endoscopic image. Also, providing the fluid guide slope 48a in the fluid ejection area brings about reduction in an area of the flat surface 36c in which the water droplets tend to build up.

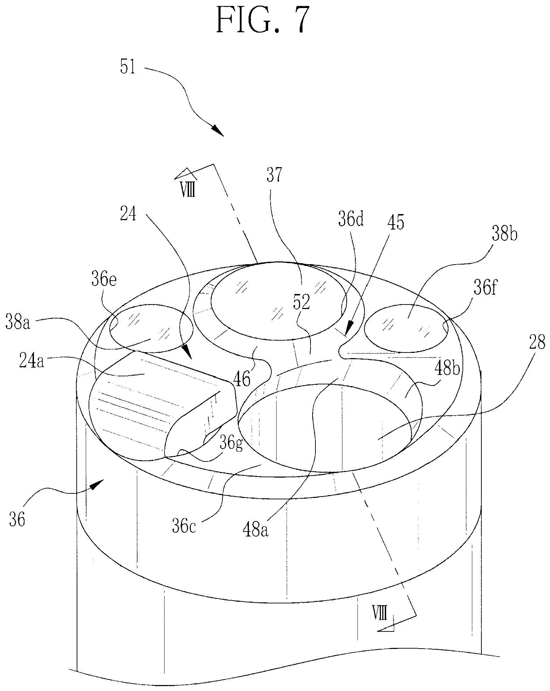

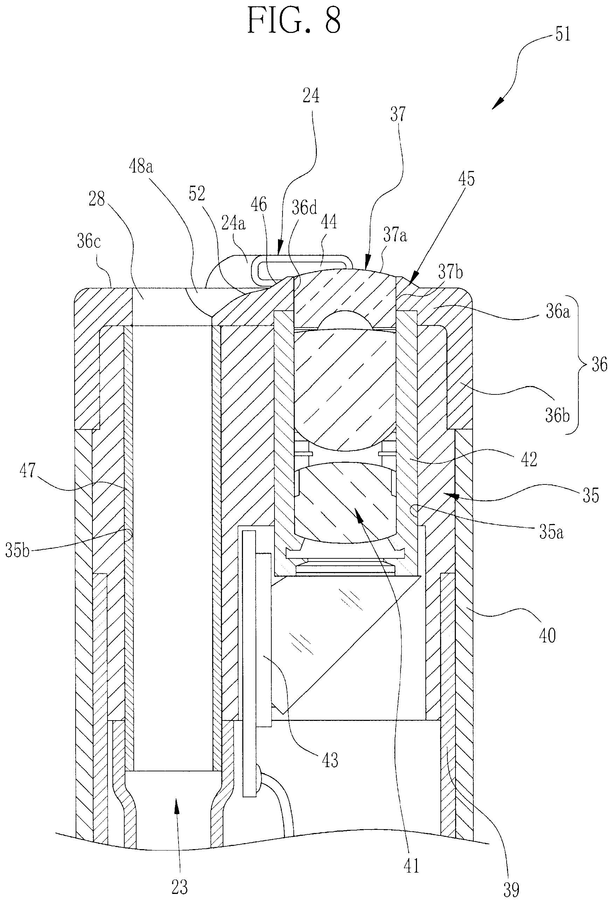

According to the above first embodiment, the flat surface 36c separates the tapered surface 46 formed around the observation window 37 from the fluid guide slope 48a, but the present invention is not limited to this. In a distal portion 51 of a second embodiment shown in Figs. 7 and 8, the tapered surface 46 and the fluid guide slope 48a may be formed continuously. In this case, the distal portion 51 has the fluid guide slopes 48a and 48b, just as with the above first embodiment. The distal portion 51 also has a joining slope 52 that extends from the fluid guide slope 48a to the tapered surface 46. The joining slope 52 is disposed along a line connecting the center of the observation window 37 and the center of the forceps outlet 28. Since all of the tapered surface 46, the joining slope 52, and the fluid guide slope 48a, which are continued, are inclined from a distal end side to a proximal end side, all water droplets adhering thereto easily flow into the forceps outlet 28, in order to facilitate further removing the water droplets ejected from the air and water nozzle 24. Note that, an inclination gradient may be same or changed from one slope to another. In the case of changing the inclination gradient, the inclination gradient may be increased or decreased.

In the distal portion 16a or 51 of each of the above embodiments, the fluid guide slopes 48a and 48b are formed continuously. However, the fluid guide slope 48b may not be formed if at least the fluid guide slope 48a is formed. Also, the entire fluid guide slopes 48a and 48b are formed in the distal end cap 36 in each of the above embodiments, but the present invention is not limited thereto. For example, a part of the distal end side of the fluid guide slopes 48a and 48b may be formed in the distal end cap 36, and the remaining part thereof may be formed in the distal portion body 35 or the connection pipe 47. In each of the above embodiments, the annular convex portion 45 formed with the tapered surface 46 is integrally formed in the distal end cap 36, but the present invention is not limited thereto. The annular convex portion 45 may be formed in a part other than the distal end cap 36, for example, the lens barrel 42.

The above embodiments take the electronic endoscope, which observes an image that takes the conditions of the object using the imaging device, as an example, but the present invention is not limited to this. The present invention is also applicable to an endoscope that observes the conditions of the object using an optical image guide.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope (10) having an insert section (16) to be introduced into a body cavity, comprising:
a flat surface (36c) formed in a distal portion (16a) of said insert section (16) in an orthogonal manner to an axial direction of said insert section (16);
an observation window (37) disposed in said flat surface (36c), for observing the inside of said body cavity;
a fluid ejection nozzle (24) disposed on said flat surface (36c), for ejecting fluid onto a surface of said observation window (37);
a lighting window (38b) positioned in an ejection area of said fluid ejected from said fluid ejection nozzle (24) and being disposed in said flat surface (36c) adjacently to said observation window (37), for applying illumination light to illuminate said inside of said body cavity; and **characterized by**
a suction port (28) disposed in said flat surface (36c) adjacently to said lighting window (38b), said observation window (37), and said fluid ejection nozzle (24), for sucking said fluid; and
a fluid guide slope (48a) disposed in said ejection area of said fluid between said lighting window (38b) and said suction port (28) so as to connect said flat surface (36c) to said suction port (28) in an inclined manner relative to said flat surface (36c), the length of said fluid guide slope (48a) from said flat surface (36c) to said suction port (28) being increased with distance from said fluid ejection nozzle (24).

2. The endoscope (10) according to claim 1, **characterized in that**
said fluid ejection nozzle (24) selectively ejects gas and liquid;
an ejection area of said gas is wider in a lateral direction than an ejection area of said liquid;
said observation window (37) is disposed in said ejection area of said liquid; and
said lighting window (38b) is disposed in said ejection area of said gas.

3. The endoscope (10) according to claim 1 or 2, **characterized in that** said fluid guide slope (48a) is the deepest relative to said flat surface (36c) at a surface in a line connecting the center of said suction port (28) to the center of said lighting window (38b) positioned in said ejection area of said fluid.

4. The endoscope (10) according to claims 1 to 3, **characterized in that** said fluid guide slope (48a) is formed so as to enlarge the internal diameter of said suction port (28) from the center of said suction port (28) to the center of said lighting window (38b).

5. The endoscope (10) according to one of claims 1 to 4, **characterized in that** said fluid guide slope (48a) is positioned at least in an area connecting the center of said suction port (28), the center of said lighting window (38b), and the center of said observation window (37).

6. The endoscope (10) according to one of claims 1 to 5, **characterized in that**
said distal portion (16a) includes a distal portion body (35) and a distal end cap (36) attached to a tip of said distal portion body (35); and
said fluid guide slope (48a) is formed in said distal end cap (36).

7. The endoscope (10) according to one of claims 1 to 6, **characterized in that** said fluid guide slope (48a) is formed along an edge of said ejection area of liquid ejected from said fluid ejection nozzle (24).

8. The endoscope (10) according to one of claims 1 to 7, **characterized in that** said fluid ejection nozzle (24) and said suction port (28) are adjacent to each other.

9. The endoscope (10) according to one of claims 1 to 8, **characterized in that** said lighting window (38b) is disposed opposite to said ejection port (44) of said fluid ejection nozzle (24) beyond said fluid guide slope (48a) in a fluid ejection direction of said fluid ejection nozzle (24).

10. The endoscope (10) according to one of claims 1 to 9, **characterized in that**
a tapered surface (46) is formed along the rim of said observation window (37), and the diameter of said tapered surface (46) is gradually reduced from said flat surface (36c) to said observation window (37); and
said tapered surface (46) and said fluid guide slope (48a) are formed continuously.

11. The endoscope (10) according to one of claims 1 to 10, **characterized in that**
a tapered surface (46) is formed along the rim of said observation window (37), and the diameter of said tapered surface (46) is gradually reduced from said flat surface (36c) to said observation window (37); and
said tapered surface (46) and said lighting window (38b) are adjacent to each other.

12. The endoscope (10) according to claims 1 to 11, **characterized in that** there is a plurality of said lighting windows (38a, 38b), and said fluid guide slope (48a) is disposed between said suction port (28) and said lighting window (38b) that is the farthest from said fluid ejection nozzle (24) and the nearest to said suction port (28).

13. The endoscope (10) according to one of claims 1 to 12, **characterized in that** said suction port (28) also functions as a forceps outlet.

## Patentansprüche

1. Endoskop (10) mit einem Einführabschnitt (16) zum Einführen in einen Körperhohlraum, umfassend:
eine in einem distalen Abschnitt (16a) des Einführabschnitts (16) ausgebildete Flachseite (36c) orthogonal zu einer axialen Richtung des Einführabschnitts (16);
ein Betrachtungsfenster (37), das in der Flachseite (36c) zum Betrachten des Inneren des Körperhohlraums angeordnet ist;
eine Fluidausstoßdüse (24), die an der Flachseite (36c) angeordnet ist, um Fluid auf eine Oberfläche des Betrachtungsfensters (37) auszustoßen;
ein Beleuchtungsfenster (38b), das in einem Ausstoßbereich des von der Fluidausstoßdüse (24) ausgestoßenen Fluids angeordnet ist und in der Flachseite (36c) benachbart zum dem Betrachtungsfenster (37) liegt, um Beleuchtungslicht zum Beleuchten des Inneren des Körperhohlraums aufzubringen, **gekennzeichnet durch**
eine Ansaugöffnung (28), die in der Flachseite (36c) benachbart zu dem Beleuchtungsfenster (38b), dem Betrachtungsfenster (37) und der Fluidausstoßdüse (24) zum Ansaugen des Fluids angeordnet ist; und
eine Fluidleitschräge (48a), die in dem Ausstoßbereich des Fluids zwischen dem Beleuchtungsfenster (38b) und der Ansaugöffnung (28) angeordnet ist, um die Flachseite (36c) mit der Saugöffnung (28) in bezüglich der Flachseite (36c) geneigter Weise zu verbinden, wobei die Länge der Fluidleitschräge (48a) von der Flachseite (36c) zu der Saugöffnung (28) mit Abstand von der Fluidausstoßdüse (24) größer wird.

2. Endoskop nach Anspruch 1, **dadurch gekennzeichnet, dass**
die Fluidausstoßdüse (24) selektiv Gas und Flüssigkeit ausstößt;
ein Ausstoßbereich des Gases in seitlicher Richtung breiter ist als ein Ausstoßbereich der Flüssigkeit;
das Betrachtungsfenster (37) in dem Ausstoßbereich der Fllüssigkeit liegt; und
das Beleuchtungsfenster (38b) in dem Ausstoßbereich des Gases liegt.

3. Endoskop (10) nach Anspruch 1 oder 2, **dadurch gekennzeichnet, dass** die Fluidleitschräge (48a) in Bezug auf die Flachseite (36c) an einer Oberfläche einer die Mitte der Saugöffnung (28) mit dem Zentrum des Beleuchtungsfensters (38b) in dem Ausstoßbereich des Fluids verbindenden Linie am tiefsten ist.

4. Endoskop (10) nach den Ansprüchen 1 bis 3, **dadurch gekennzeichnet, dass** die Fluidleitschräge (48a) derart ausgebildet ist, dass sich der Innendurchmesser der Saugöffnung (28) ausgehend von der Mitte der Saugöffnung (28) hin zum Zentrum des Beleuchtungsfensters (38b) vergrößert.

5. Endoskop (10) nach einem der Ansprüche 1 bis 4, **dadurch gekennzeichnet, dass** die Fluidleitschräge (48a) zumindest in einem Bereich gelegen ist, der das Zentrum der Saugöffnung (28), das Zentrum des Beleuchtungsfensters (38b) und das Zentrum des Betrachtungsfensters (37) verbindet.

6. Endoskop (10) nach einem der Ansprüche 1 bis 5, **dadurch gekennzeichnet, dass**
der distale Abschnitt (16a) einen Distalabschnittskörper (35) und eine Distalendkappe (36), die am Ende des Distalabschnittskörpers (35) angebracht ist, enthält; und
die Fluidleitschräge (48a) in der Distalendkappe (36) ausgebildet ist.

7. Endoskop (10) nach einem der Ansprüche 1 bis 6, **dadurch gekennzeichnet, dass** die Fluidleitschräge (48a) entlang einem Rand des Ausstoßbereichs der von der Fluidausstoßdüse (24) ausgestoßenen Flüssigkeit ausgebildet ist.

8. Endoskop (10) nach einem der Ansprüche 1 bis 7, **dadurch gekennzeichnet, dass** die Fluidausstoßdüse (24) und die Ansaugöffnung (28) einander benachbart sind.

9. Endoskop (10) nach einem der Ansprüche 1 bis 8, **dadurch gekennzeichnet, dass** das Beleuchtungsfenster (38b) sich gegenüber der Ausstoßöffnung (44) der Fluidausstoßdüse (24) jenseits der Fluidleitschräge (48a) in Fluidausstoßrichtung der Fluidausstoßdüse (24) befindet.

10. Endoskop (10) nach einem der Ansprüche 1 bis 9, **dadurch gekennzeichnet, dass**
eine verjüngte Fläche (46) entlang dem Rand des Betrachtungsfensters (37) gebildet ist, und der Durchmesser der verjüngten Fläche (46) ausgehend von der Flachseite (36c) zu dem Betrachtungsfenster (37) hin allmählich verkleinert ist; und
die verjüngte Fläche (46) und die Fluidleitschräge (48a) kontinuierlich ausgebildet sind.

11. Endoskop (10) nach einem der Ansprüche 1 bis 10, **dadurch gekennzeichnet, dass**
eine verjüngte Fläche (46) entlang dem Rand des Betrachtungsfensters (37) ausgebildet ist, und der Durchmesser der verjüngten Fläche (46) ausgehend von der Flachseite (36c) hin zu dem Betrachtungsfenster (37) allmählich verkleinert ist; und
die verjüngte Fläche (46) und das Beleuchtungsfenster (38b) einander benachbart sind.

12. Endoskop (10) nach einem der Ansprüche 1 bis 11, **dadurch gekennzeichnet, dass** es mehrere Beleuchtungsfenster (38a, 38b) gibt, und die Fluidleitschräge (48a) sich zwischen der Saugöffnung (28) und dem Beleuchtungsfenster (38b), das von der Fluidausstoßdüse (24) am weitesten entfernt ist und der Saugöffnung (28) am nächsten liegt, angeordnet ist.

13. Endoskop (10) nach einem der Ansprüche 1 bis 12, **dadurch gekennzeichnet, dass** die Saugöffnung (28) als Zangenauslass fungiert.

## Revendications

1. Endoscope (10) présentant une section d'insert (16) à introduire dans une cavité corporelle, comprenant :
une surface plate (36c) formée dans une portion distale (16a) de ladite section d'insert (16) de manière orthogonale par rapport à une direction axiale de ladite section d'insert (16) ;
une fenêtre d'observation (37) disposée dans ladite surface plate (36c), destinée à observer l'intérieur de ladite cavité corporelle ;
une buse d'éjection de fluide (24) disposée sur ladite surface plate (36c), destinée à éjecter un fluide sur une surface de ladite fenêtre d'observation (37) ;
une fenêtre d'éclairage (38b) positionnée dans une zone d'éjection dudit fluide éjecté à partir de ladite buse d'éjection de fluide (24) et disposée dans ladite surface plate (36c) de manière adjacente à ladite fenêtre d'observation (37), destinée à appliquer une lumière d'éclairage pour éclairer ledit intérieur de ladite cavité corporelle, et **caractérisé par**
un orifice d'aspiration (28) disposé dans ladite surface plate (36c) de manière adjacente à ladite fenêtre d'éclairage (38b), ladite fenêtre d'observation (37), et ladite buse d'éjection de fluide (24), pour aspirer ledit fluide, et
une pente de guidage de fluide (48a) disposée dans ladite zone d'éjection dudit fluide entre ladite fenêtre d'éclairage (38b) et ledit orifice d'aspiration (28) de manière à relier ladite surface plate (36c) audit orifice d'aspiration (28) d'une manière inclinée par rapport à ladite surface plate (36c), la longueur de ladite pente de guidage de fluide (48a) de ladite surface plate (36c) audit orifice d'aspiration (28) étant augmentée avec la distance à partir de ladite buse d'éjection de fluide (24).

2. Endoscope (10) selon la revendication 1, **caractérisé en ce que**
ladite buse d'éjection de fluide (24) éjecte de manière sélective du gaz et du liquide ; une zone d'éjection dudit gaz est plus large dans une direction latérale qu'une zone d'éjection dudit liquide ;
ladite fenêtre d'observation (37) est disposée dans ladite zone d'éjection dudit liquide, et
ladite fenêtre d'éclairage (38b) est disposée dans ladite zone d'éjection dudit gaz.

3. Endoscope (10) selon la revendication 1 ou 2, **caractérisé en ce que** ladite pente de guidage de fluide (48a) est la plus profonde par rapport à ladite surface plate (36c) sur une surface en ligne reliant le centre dudit orifice d'aspiration (28) au centre de ladite fenêtre d'éclairage (38b) positionnée dans ladite zone d'éjection dudit fluide.

4. Endoscope (10) selon l'une quelconque des revendications 1 à 3, **caractérisé en ce que** ladite pente de guidage de fluide (48a) est formée de manière à agrandir le diamètre interne dudit orifice d'aspiration (28) du centre dudit orifice d'aspiration (28) au centre de ladite fenêtre d'éclairage (38b).

5. Endoscope (10) selon l'une quelconque des revendications 1 à 4, **caractérisé en ce que** ladite pente de guidage de fluide (48a) est positionnée au moins dans une zone reliant le centre dudit orifice d'aspiration (28), le centre de ladite fenêtre d'éclairage (38b), et le centre de ladite fenêtre d'observation (37).

6. Endoscope (10) selon l'une quelconque des revendications 1 à 5, **caractérisé en ce que** ladite portion distale (16a) inclut un corps de portion distale (35) et un chapeau d'extrémité distale (36) attaché à un bout dudit corps de portion distale (35), et ladite pente de guidage de fluide (48a) est formée dans ledit chapeau d'extrémité distale (36).

7. Endoscope (10) selon l'une quelconque des revendications 1 à 6, **caractérisé en ce que** ladite pente de guidage de fluide (48a) est formée le long d'un bord de ladite zone d'éjection du liquide éjecté à partir de ladite buse d'éjection de fluide (24).

8. Endoscope (10) selon l'une quelconque des revendications 1 à 7, **caractérisé en ce que** ladite buse d'éjection de fluide (24) et ledit orifice d'aspiration (28) sont adjacents entre eux.

9. Endoscope (10) selon l'une quelconque des revendications 1 à 8, **caractérisé en ce que** ladite fenêtre d'éclairage (38b) est disposée face audit orifice d'éjection (44) de ladite buse d'éjection de fluide (24) au-delà de ladite pente de guidage de fluide (48a) dans une direction d'éjection de fluide de ladite buse d'éjection de fluide (24).

10. Endoscope (10) selon l'une quelconque des revendications 1 à 9, **caractérisé en ce que**
une surface de diminution progressive (46) est formée le long d'un rebord de ladite fenêtre d'observation (37), et le diamètre de ladite surface de diminution progressive (46) est graduellement réduit de ladite surface plate (36c) à ladite fenêtre d'observation (37), et
ladite surface de diminution progressive (46) et ladite pente de guidage de fluide (48a) sont formées de manière continue.

11. Endoscope (10) selon l'une quelconque des revendications 1 à 10, **caractérisé en ce que**
une surface de diminution progressive (46) est formée le long d'un rebord de ladite fenêtre d'observation (37), et le diamètre de ladite surface de diminution progressive (46) est graduellement réduit de ladite surface plate (36c) à ladite fenêtre d'observation (37), et
ladite surface de diminution progressive (46) et ladite fenêtre d'éclairage (38b) sont adjacentes entre elles.

12. Endoscope (10) selon l'une quelconque des revendications 1 à 11, **caractérisé en ce qu'**il existe une pluralité desdites fenêtres d'éclairage (38a, 38b), et ladite pente de guidage de fluide (48a) est disposée entre ledit orifice d'aspiration (28) et ladite fenêtre d'éclairage (38b) qui est la plus éloignée de ladite buse d'éjection de fluide (24) et la plus proche dudit orifice d'aspiration (28).

13. Endoscope (10) selon l'une quelconque des revendications 1 à 12, **caractérisé en ce que** ledit orifice d'aspiration (28) sert également d'orifice de sortie de davier.
